# EUROPEAN PATENT APPLICATION

(11) **EP 4 235 180 A1**
(43) Date of publication of application: **30.08.2023**
(21) Application number: 22305216.8
(22) Date of filing: 25.02.2022
(51) Int. Cl.: G01N 33/68

(54) **URINARY METABOLOMIC PROFILING IN TOLERANT KIDNEY TRANSPLANT RECIPIENTS**

(71) Applicant: Nantes Université, 44000 Nantes (FR); Institut National de la Santé et de la Recherche Médicale, 75013 Paris (FR); Chu Nantes, 44000 Nantes (FR)
(72) Inventor: BROUARD, Sophie, 44240 Sucé sur Erdre (FR); COLAS, Luc, 44850 Le Cellier (FR)
(74) Representative: Novagraaf Technologies

(57) **Abstract**

The present invention concerns a new urinary metabolomic signature of spontaneous operational tolerance (SOT) in kidney transplant recipients/patients (KTR) showing enrichment in tryptophan-derived metabolites.

## Description

### DESCRIPTION

### Technical field of the invention

The present invention concerns a new urinary metabolomic signature of spontaneous operational tolerance (SOT) or low risk of rejection (LRR), in kidney transplant recipients/patients (KTR) with a long-term functional kidney independent of immunosuppressive drugs, showing enrichment in tryptophan-derived metabolites.

In the description below, references in square brackets ([ ]) refer to the list of references at the end of the text.

### State of the art

Kidney failure is defined as the partial or total loss of the ability of the kidneys to filter the blood to remove waste products such as urea and creatinine. In kidney failure, urine becomes more concentrated and the amount of waste products eliminated in the same volume decreases. The measurement of blood creatinine and urea is one way of revealing renal failure. The urine directly produced by kidney and urinary compartment are easily accessible, and thus potentially reflects homeostatic or pathologic mechanisms in the kidney organ.

Despite the evolution of techniques in the field of organ transplantation as well as the evolution of immunosuppressive drugs, the incidence of chronic rejection (long-term loss of graft function) remains very high.

Metabolomic is a recent growing field allowing to depict spatiotemporal shifts in metabolic pathways associated with homeostatic or pathologic processes. Metabolomic is a new non-invasive tool for the measurement of endogenous metabolites formed as end products or intermediates of metabolic pathways and collected in patient samples such as blood, urine, saliva, etc. In the field of kidney transplantation, a few publications have associated metabolic changes with graft rejection in solid organ transplant immunology [1-5]. In those studies, tryptophan-derived metabolites were detected (decreased) either in blood or urine compartments suggesting that the kynurenine pathway may be involved in kidney allograft alloimmune damages. Tryptophan is decreased and correlated with estimated Glomerular Filtration Rate (eGFR) in the serum of patients with chronic allograft dysfunction compared to stable KTR and healthy volunteers (HV) [2]. In urine, a recent study by Sigdel TK et al associated three main metabolites (increase in glycine, decrease in N-methylalanine and inulobiose) in the urine with histology proven heterogenous allo-immune dysfunctions of kidney transplants (acute rejections and IFTA) [6]. Interestingly, glycine has been associated with broad anti-inflammatory properties, ischemia/reperfusion and thermic shock protection [7]. On the opposite, N-methylalanine was negatively correlated with active inflammation in patients with rheumatoid arthritis [8]. At last, inulobiose is an exogenous metabolite derived from inuline which is not reabsorbed after glomerular filtration [9]. The difference in urine inulobiose concentration during allo-immune dysfunction could be linked to a lower eGFR. Interestingly, no tryptophane derived metabolites were highlighted. Conversely, tryptophan was decreased in patients with T-cell mediated rejection (TCMR) compared with stable KTR under a standard immunosuppressive drug regimen [1,4] or KTR with Antibody Mediated Rejection (ABMR) [5]. Moreover, Duranton et al, reported on amino acid variations in the plasma and urine of patients with chronic kidney disease (CKD) and hemodialyzed patients. In particular, tryptophan and hydroxykynurenine (direct down-stream kynurenine metabolite) are modulated and associated with eGFR variations in the plasma, whereas no variation was observed in the urine of the same patients [10]. Similarly, Goek et al also demonstrated that the kynurenine pathway was upregulated by eGFR impairment in the plasma but not in urine [11] whereas urinary excretion of serotonin, another tryptophan-derived metabolite, was lower in KTR with eGFR impairment [3]. At last, Bassi et al reported that no difference in urine tryptophan concentration according to eGFR in KTR with allograft dysfunction [2]. These observations suggest that 1) kynurenine pathway deviation may be associated with kidney transplant stability and low risk of rejection (either with immunosuppressive (IS) drugs or SOT); 2) eGFR impairment has a different impact on the tryptophan metabolic pathways in plasma and urine. These preliminary results suggest that the immunological state and kidney graft function could be monitored at locoregional level in a non-invasive fashion using targeted metabolomic. Though, it should be born in mind that metabolomic techniques can skew the metabolomic profile [12].

Spontaneous operational tolerance (SOT) in kidney transplantation (KTx) is defined as stable graft function in a recipient off immunosuppressive drugs and in whom no clinically significant detrimental immune responses and immune deficits are detected. Thus, SOT can be associated with a low risk of rejection (LRR) [13,14]. It is a rare state although it is likely more frequent than induced tolerance thanks to haploidentical hematopoietic stem cell (HSCT) and kidney transplantation [15]. Two cohorts of patients with SOT showed specific clinical features such as being for at least 2/3 of male gender with low even null rate of rejection, no donor specific antibodies and antibiotic [16,17]. Additionally, they present specific immune features such as higher systemic rate of granzyme B positive regulatory B cells [18] and memory regulatory T cells [19] and defective NK [20] and Tfh cells function [21]. All these immune features makes an echo to transcriptomic analysis of their peripheral blood mononuclear cells (PBMCs) that exhibited a regulatory T [13] and B cell profiles [13,17]. More recently, SOT was demonstrated to be associated with a unique and specific urinary proteobacteria signature [22] suggesting that a functional interplay between this specific microbiota and KTR immunoregulatory mechanisms potentially associated with a specific metabolome at a locoregional level.

However to date, no metabolomic signature associated with SOT or LRR in human renal transplantation has been developed.

### Description of the invention

The Inventors now demonstrated for the first time that spontaneous operational tolerance (SOT) or low risk of rejection (LRR) in kidney transplant patients/recipients (KTR) was associated with a specific urinary metabolomic signature enriched in tryptophan-derived metabolites from the kynurenine pathway allowing to characterize tolerant patients (TOL) with a high sensitivity and specificity.

To this aim, they performed a metabolomic profiling via ultrahigh performance liquid chromatography in tandem with mass spectrometry (UHPLC/MS) in the urine samples of patients having SOT (TOL) compared with stable KTR (STA), minimally immunosuppressed KTR (MIS) and healthy volunteers (HV) (i.e. without chronic inflammatory diseases or chronic renal disease). Supervised and unsupervised multivariate computational analyses were used to highlight urinary metabolomic profile and metabolite identification thanks to workflow4metabolomic platform.

The Inventors found that the urinary metabolome was composed of approximately 2700 metabolites. Raw unsupervised clustering allowed to separate healthy volunteers and patients having SOT from others. For the first time, they clearly identified a specific urinary metabolomic signature associated with patients having SOT encompassing a high concentration of tryptophan-derived metabolites from the kynurenine pathway mainly driven by kynurenic acid and tryptamine. Said metabolomic profile is independent of serum creatinine level whereas the serotonin pathway was downregulated by high serum creatinine levels (which is consistent with the aforementioned literature [10,11]). No association between the immunosuppressive regimen and tryptophan-derived metabolites (kyrunenine, trypyamine or serotonin/melatonin pathways) was observed either, in accordance with data in rodents and human KTR despite difference in metabolomic techniques [12]. No association with donor-specific antibodies or the gender of the recipient was observed either.

Altogether, the results argue for an upregulation of the kynurenine (i.e. kynurenine and kynurenic acid) and the tryptamine pathways in SOT patients compared to HV and kidney transplanted recipients with stable graft function under standard immunosuppressive regimen (STA) or minimal immunosuppressive regimen (MIS) that were grouped as non tolerant (nonTOL), suggesting their implication in spontaneous operational tolerance (SOT) or low risk or rejection (LRR) state independent of eGFR and immunosuppressive regimen. The results also suggested that kynurenine, kynurenic acid and tryptamine enrichment in the urine of TOL allowed the identification of putative pathways and metabolites associated with spontaneous operational tolerance such as Indole 2,3-diamine oxygenase (IDO), GRP35, Aryl Hydrocarbon Receptor (AhR) signaling and microbiota-derived tryptophan metabolites such as indole alkaloids.

Therefore an object of the present invention concerns an *in vitro* method for determining a spontaneous operational tolerance (SOT) and/or low risk of rejection (LRR) profile in a kidney transplanted recipient (KTR), said method comprising:
a) determining in a urine sample of said KTR the expression level of at least one tryptophan-derived metabolite in said sample;
b) comparing the expression level detected in step a) to a reference value of said at least one tryptophan-derived metabolite;
c) deducing from said comparison that the KTR has a SOT and/or LRR profile when the expression level of at least one tryptophan-derived metabolite detected in step a) is equal to or higher than the reference value of said at least one tryptophan-derived metabolite.

According to a particular embodiment of the method of the present invention, the urine sample may be composed of one or more urine samples which are homogenised and centrifuged.

According to a particular embodiment of the method of the present invention, the step of detection a) is carried out by ultrahigh performance liquid chromatography and/or hydrophilic Interaction liquid chromatography in tandem with mass spectrometry (HILIC UHPLC/MS).

According to a particular embodiment of the method of the present invention, step a) detects the expression level of at least one tryptophan-derived metabolite chosen from the group consisting of kynurenic acid, kynurenine, and tryptamine. Preferably step a) detects the expression level of kynurenic acid.

According to a particular embodiment of the method of the present invention, the detection of the expression level of kynurenic acid comprises the detection of one or more ions chosen from the group consisting of ions M144T186, M188T186, M190T186 and M191T186.

According to a particular embodiment of the method of the present invention, the detection of the expression level of kynurenine comprises the detection of ions M209T8 and/or M207T89.

According to a particular embodiment of the method of the present invention, the detection of the expression level of tryptamine comprises the detection of ions M144T259.

According to a particular embodiment of the method of the present invention, the reference value of at least one tryptophan-derived metabolite in step b) is the expression level of said at least one tryptophan-derived metabolite in a urine sample of a KTR having a SOT or LRR profile.

The present invention also relates to a kit-of-parts for performing (or implementing) the method of the present invention, said kit-of-parts comprising means for determining the expression level of at least one tryptophan-derived metabolites, preferably selected from the group consisting of kynurenic acid, kynurenine and tryptamine. For example, the kit-of-parts comprises at least one means for absolute quantification of at least one tryptophan-derived metabolites, preferably selected from the group consisting of kynurenic acid, kynurenine and tryptamine, and optionnaly using isotopically labelled internal standard. Such means may be based, e.g., on 6500+ Q-Trap LC/MS system.

As used herein, the term "kit-of-parts" refers to an article of manufacture comprising at least one means for determining the expression level of at least one tryptophan-derived metabolites, preferably selected from the group consisting of kynurenic acid, kynurenine and tryptamine. Such means may be, e.g., apparatus adapted for ultrahigh performance liquid chromatography with high-resolution mass spectrometry (UHLPC/MS), or apparatus adapted for Hydrophilic Interaction Liquid Chromatography (HILIC UHPLC-MS).

According to a particular embodiment of the kit-of-parts of the invention, means for determining the expression level of kynurenic acid comprises means for the detection of one or more ions chosen from the group consisting of ions M144T186, M188T186, M190T186 and M191T186.

According to a particular embodiment of the kit-of-parts of the invention, means for determining the expression level of kynurenine comprises means for the detection of ions M209T89 and/or M207T89.

According to a particular embodiment of the kit-of-parts of the invention, means for determining the expression level of tryptamine comprises means for the detection of ions M144T259.

### Brief description of the figures

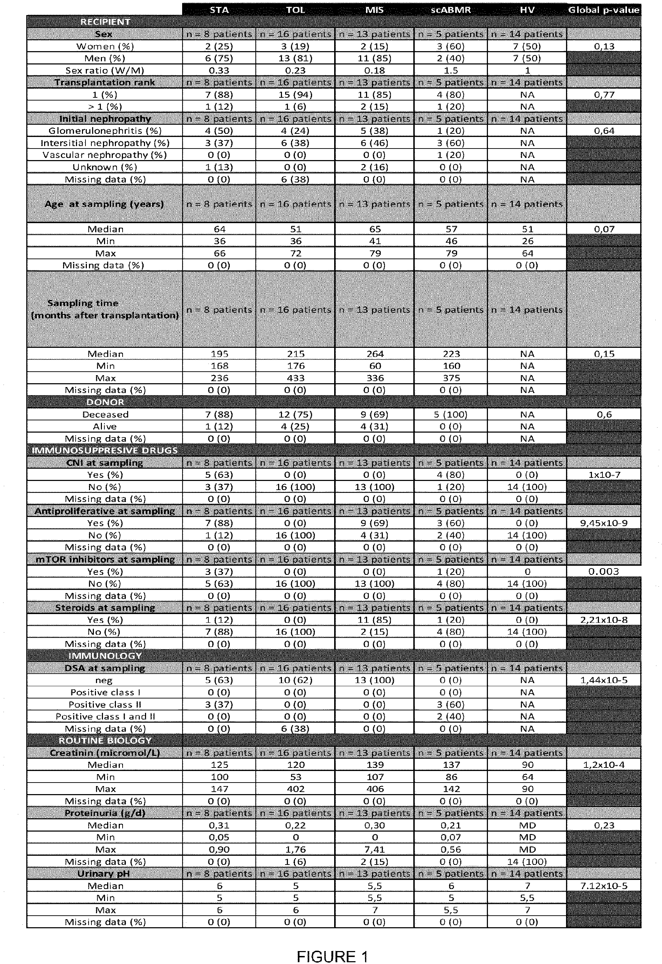

Figure 1 is a table representing clinical and biological characteristics of recipient groups (TOL, MIS, STA, scABMR) and healthy volunteers. * indicates significant adjusted p-value < 0.05 and NA indicates "not applicable".

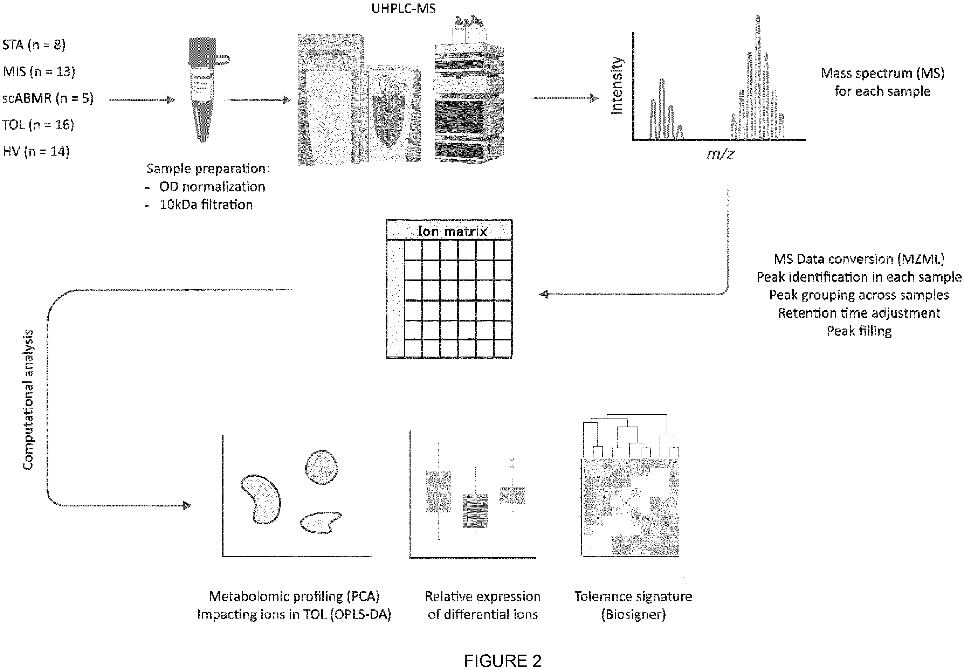

Figure 2 is a schematic representation of the workflow used to identify the metabolomic signature of spontaneous operational tolerance. This figure was created by BioRender.

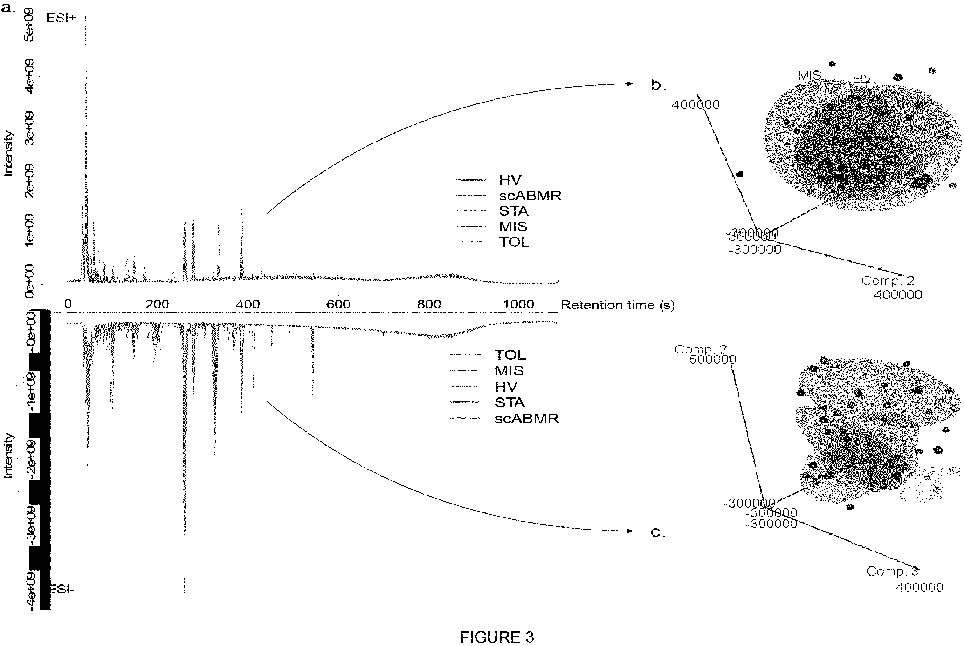

Figure 3 represents richness and structure of the urinary metabolome for each group of KTR and HV with Reversed Phase (RP) UHPLC-MS method. (a) Chromatogram showing showing 2161 ions and 2681 ions with a major proportion of highly polar and polar metabolites in ElectroSpary lonozation positive (ESI+) and ElectroSpary lonozation negative (ESI-) modes respectively according acetonitrile gradient and retention time (RT). (b) Structure of urinary metabolome in ESI+ assessed by principal component analysis (PCA) with the first three components for recipient groups (TOL, MIS, STA, scABMR) and healthy volunteers revealing two clusters: one grouping TOL and HV and another grouping MIS, STA and scABMR. (c) Structure of the urinary metabolome in ESI- assessed by principal component analysis (PCA) with the first three components for recipient groups (TOL, MIS, STA, scABMR) and healthy volunteers revealing an isolated cluster of HV in the three principal components (PCs) and KTR clustering in roughly parallel planes from TOL/MIS/STA/scABMR.

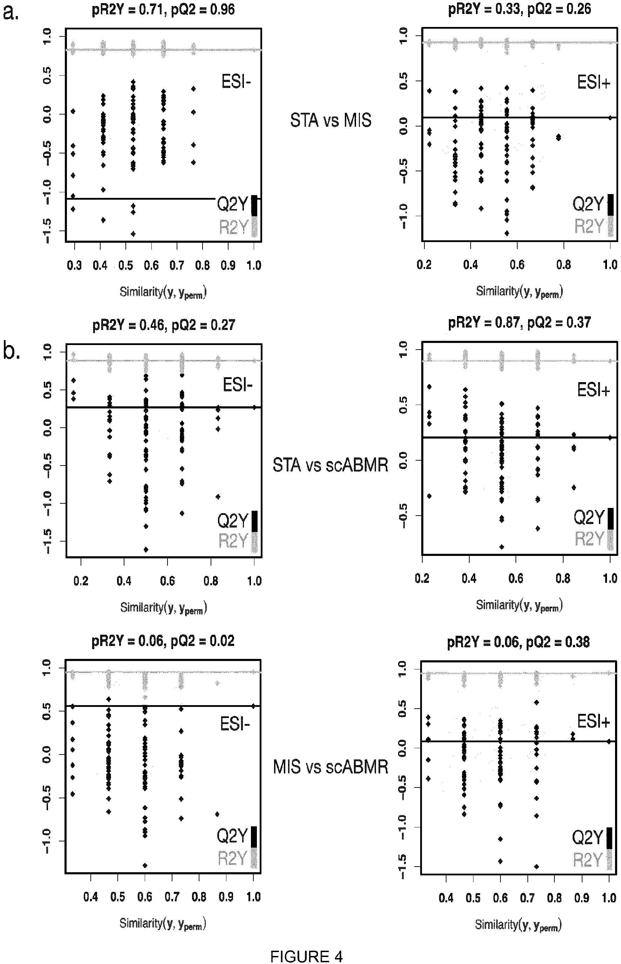

Figure 4 represents permutation diagrams of OPLS-DA for (a) MIS versus STA in both modes and (b) scABMR versus MIS or STA in both modes.

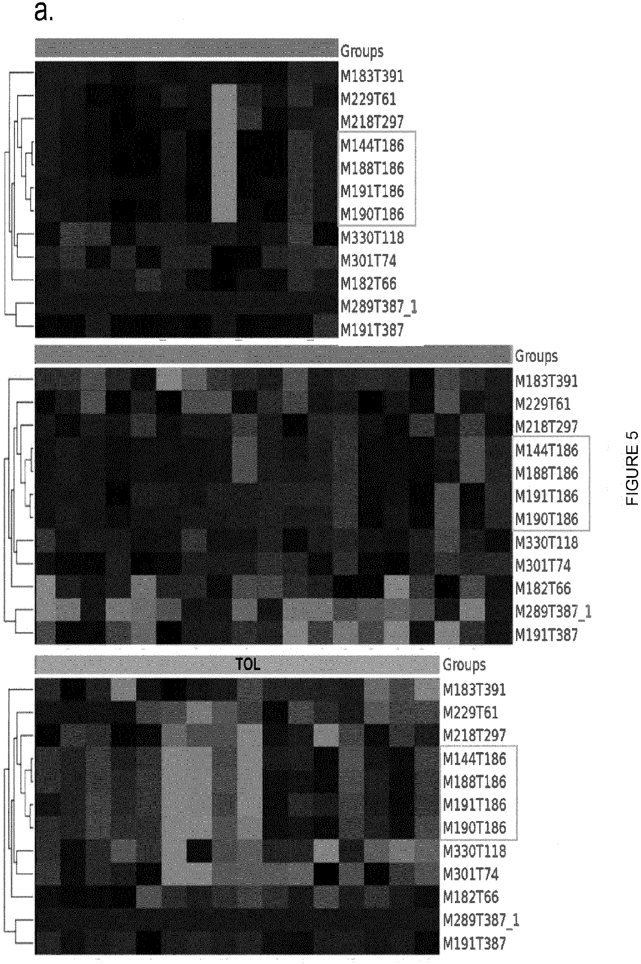

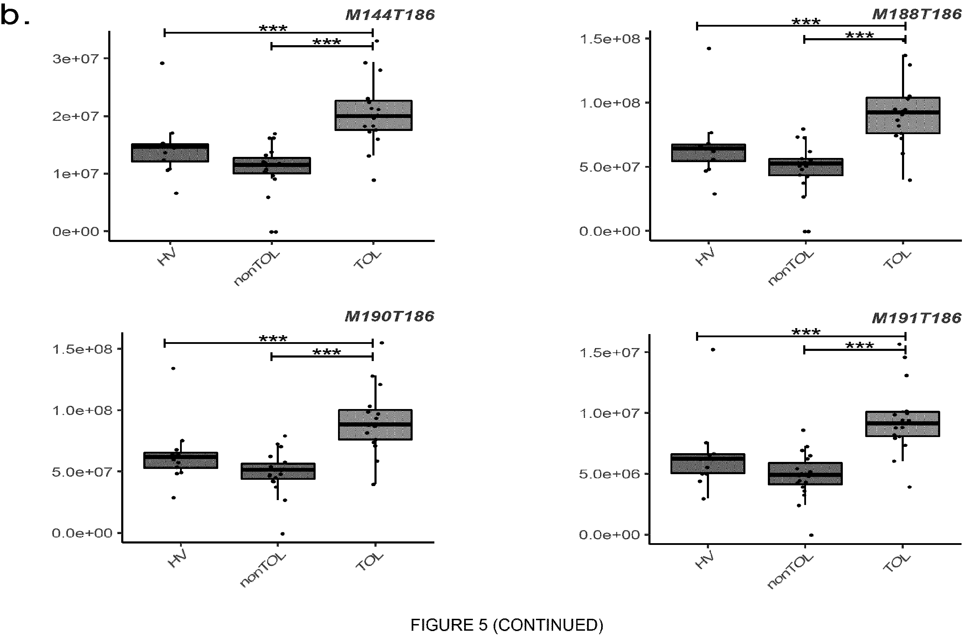

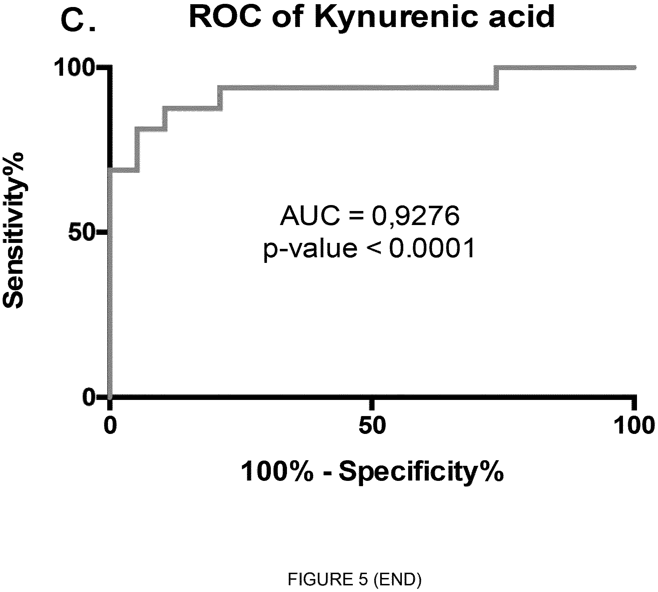

Figure 5 represents the specific metabolomic signature in urine of TOL detected thanks to RP UHPLC-MS method. (a) represents the supervised clustered heatmap according to KTR (TOL, nonTOL) and HV of the twelve ions composing the specific urinary signature of TOL patients where ten are upregulated in TOL (middle to light grey) and two are downregulated in TOL (black to dark grey). Among the twelve ions, four were identified as being adducts of kynurenic acid (highlighted in red) (b) as shown in boxplots (c) which allow a good discrimination of TOL compared to nonTOL patients according to the Receiver Opertating Characteristics Curve (ROCC). * indicates a False Discover Rate (FDR)-adjusted p-value < 0.1 ; ** indicates an FDR-adjusted p-value < 0.01 and ***indicates an FDR-adjusted p-value < 0.001.

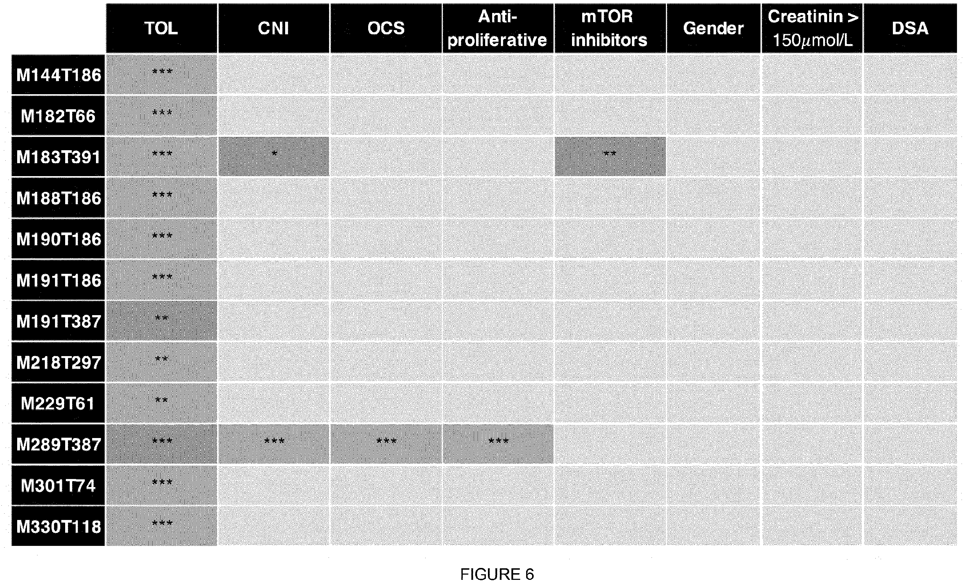

Figure 6 is a table representing the interaction matrix of the urinary metabolomic signature of TOL detected thanks to RP UHPLC-MS method. Each column represents a tested factor among the interaction models (N-way ANOVA) for each of the twelve ions identified (in rows). A color code features either the upregulation (middle grey) or the downregulation (dark grey) or the absence of change (light grey) induced by the considered factor. No interaction with the tested factors was detected for kynurenic acid. There was also an inverse interaction between two ions and immunosuppressive drugs when considering TOL. The statistical significance of the interaction is represented by * indicating an FDR-adjusted p-value < 0.1 ; ** indicating an FDR-adjusted p-value < 0.01 and *** indicating an FDR-adjusted p-value < 0.001.

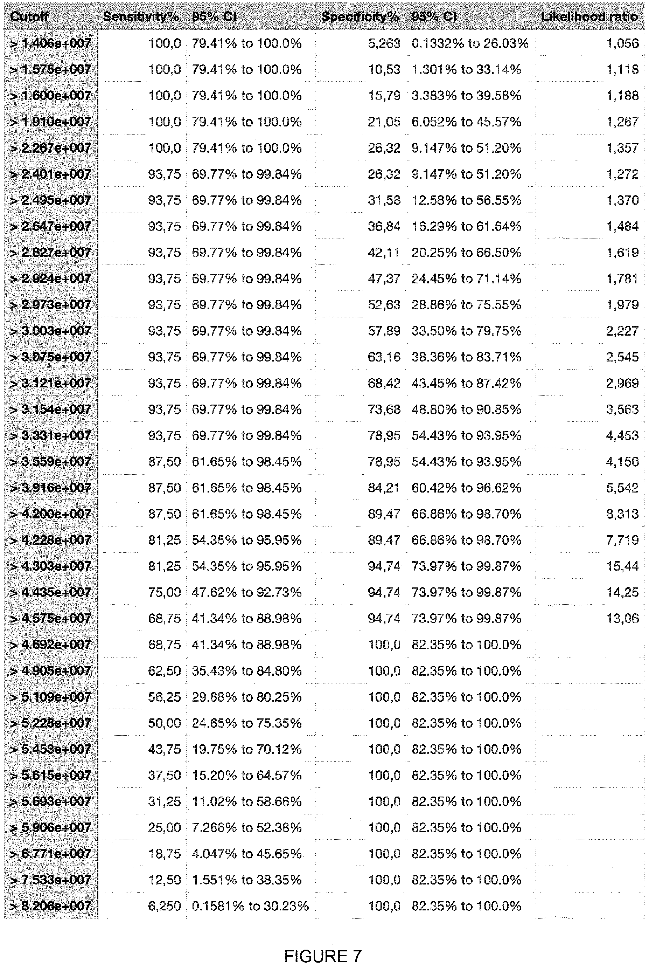

Figure 7 represents the sensitivity and specificity table of ROCC of kynurenic acid (TOL versus nonTOL) with the likehood ratio allowing us to determine the best cutoff value to discriminate TOL.

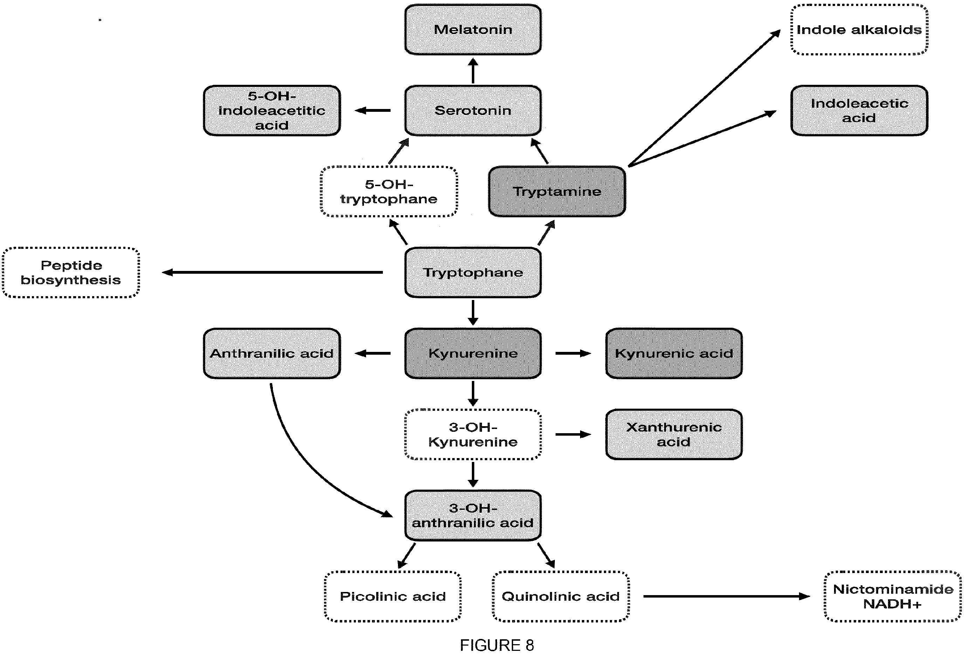

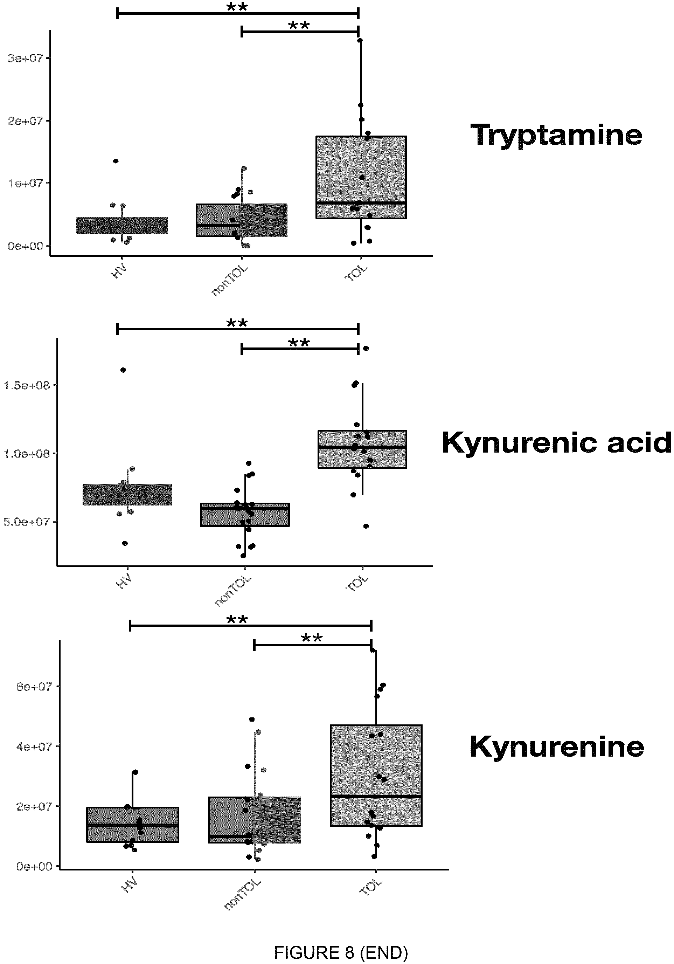

Figure 8 represents tryptophan-derived metabolites detected in the urine samples of our cohort (TOL, nonTOL and HV) and their associated metabolic pathways detected thanks to RP UHPLC-MS method. Kynurenine, kynurenic acids and tryptamine were upregulated in TOL compared to nonTOL and HV as shown in boxplots. Solid lines represent detected and identified metabolites ; dashed lines represent nonidentified metabolites ; light grey shading indicates no change in TOL ; dark grey shading indicates downregulation ; middle grey shading indicates upregulation ; * indicates an FDR-adjusted p-value < 0.1 ; ** indicates an FDR-adjusted p-value < 0.01 and *** indicates an FDR-adjusted p-value < 0.001.

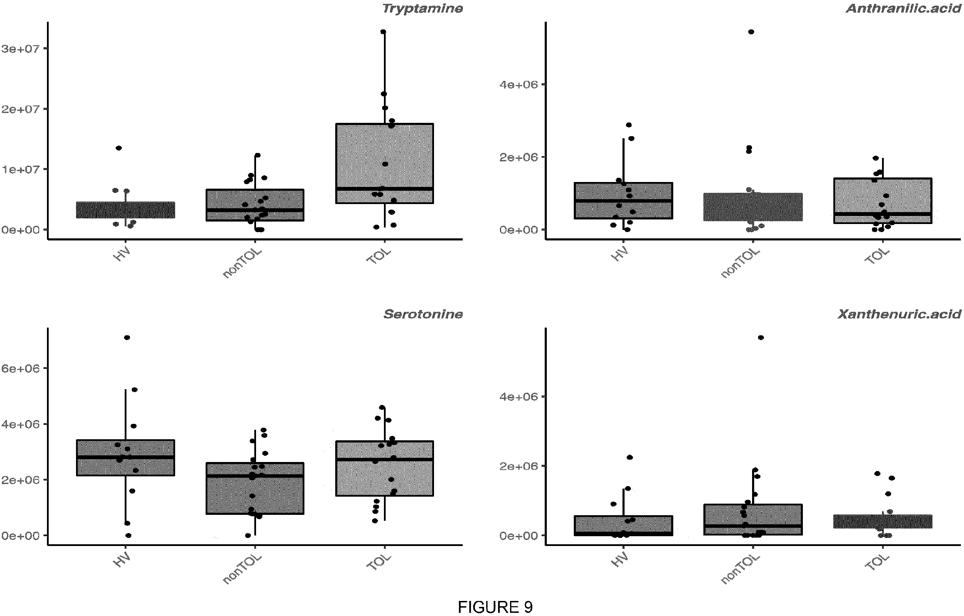

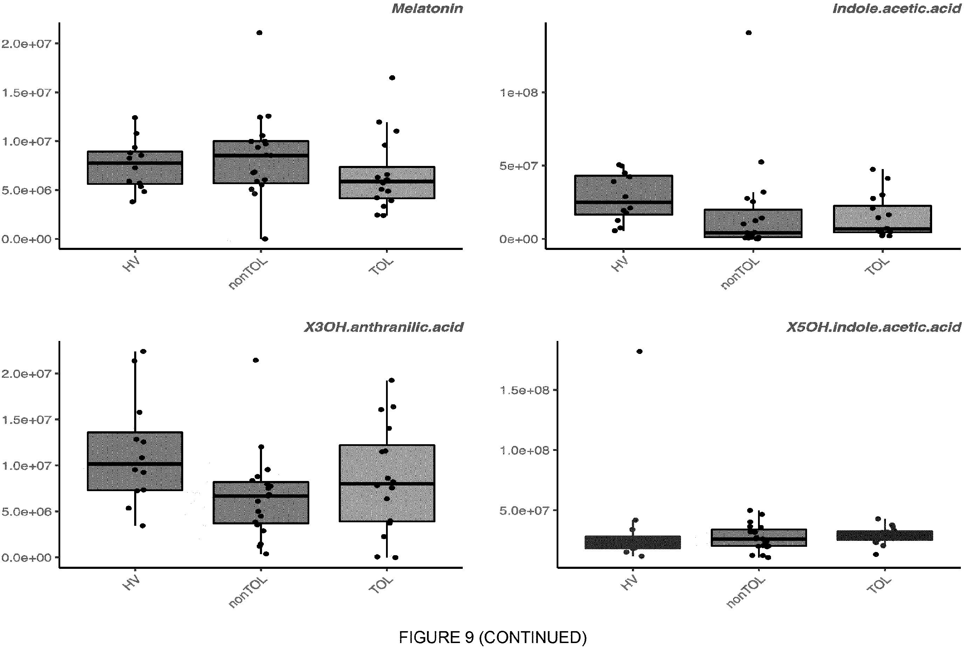

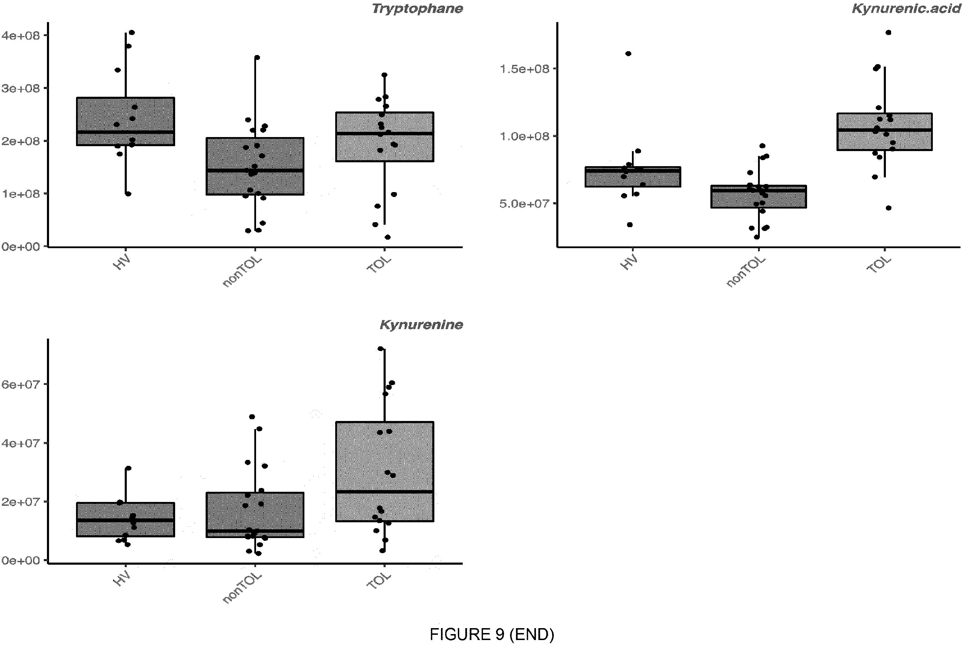

Figure 9 represents boxplots of the eleven tryptophan-derived metabolites identified and detected in the urinary sample of the cohort (TOL, nonTOL and HV) thanks to RP UHPLC-MS method. * indicates an FDR-adjusted p-value < 0.1 ; ** indicates an FDR-adjusted p-value < 0.01 and *** indicates an FDR-adjusted p-value < 0.001.

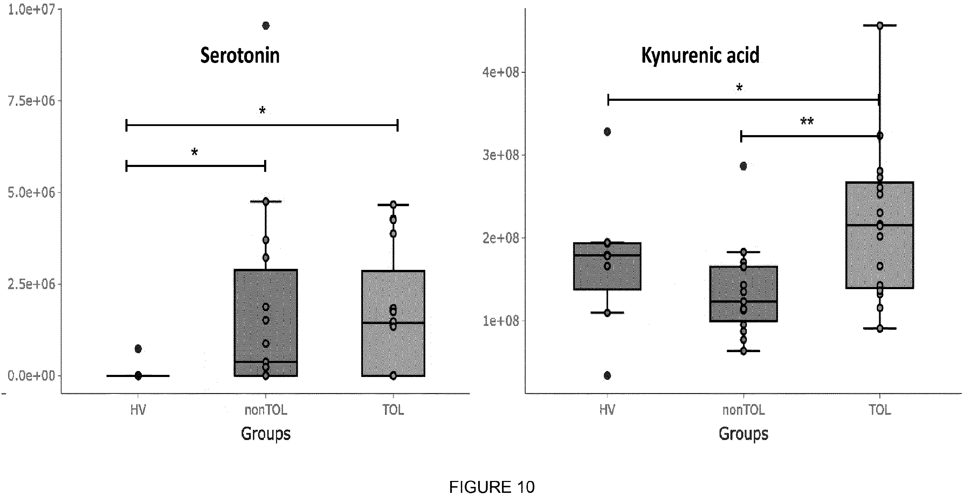

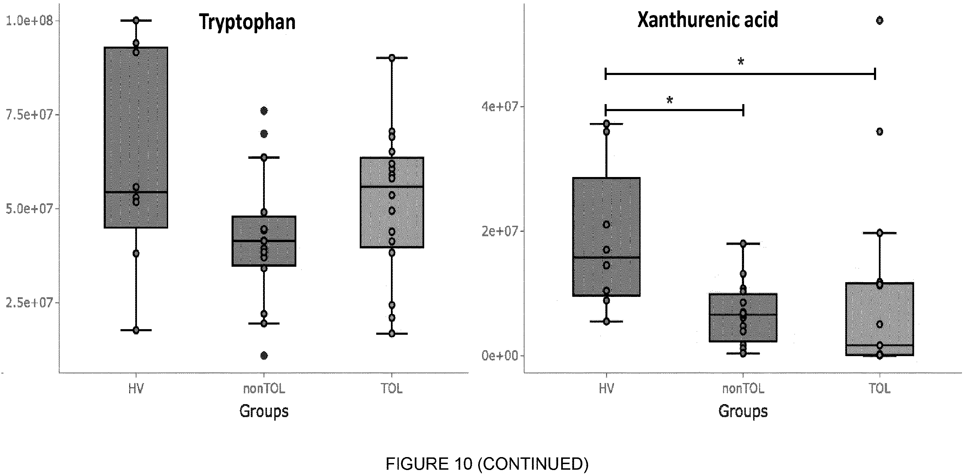

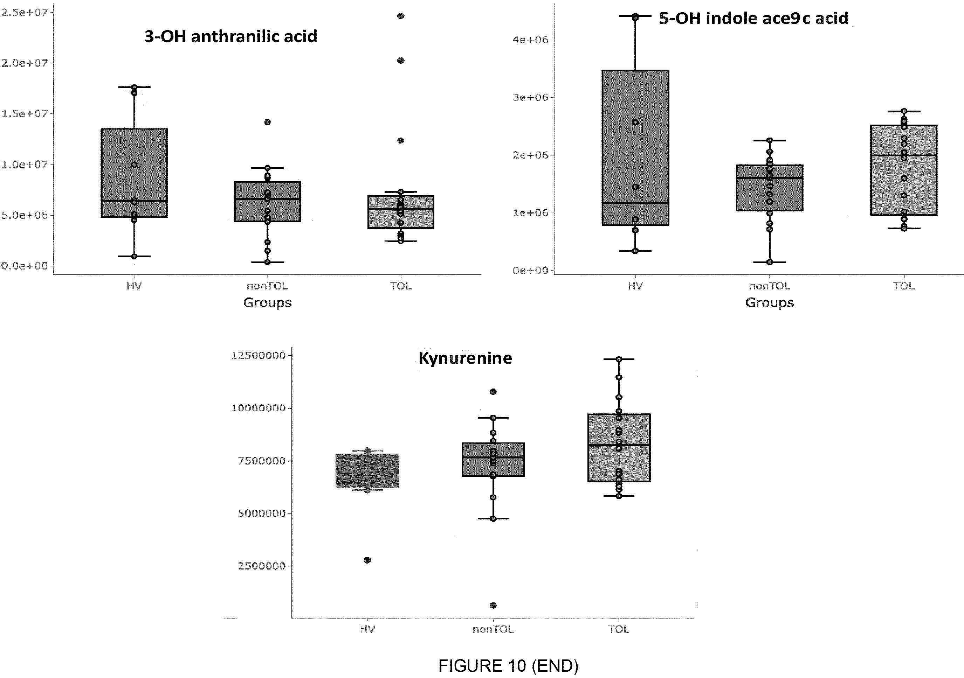

Figure 10 represents boxplots of the seven tryptophan-derived metabolites identified and detected in the urinary sample of the cohort (TOL, nonTOL and HV) with HILIC UHPLC-MS method. * indicates an FDR-adjusted p-value < 0.1 ; ** indicates an FDR-adjusted p-value < 0.01.

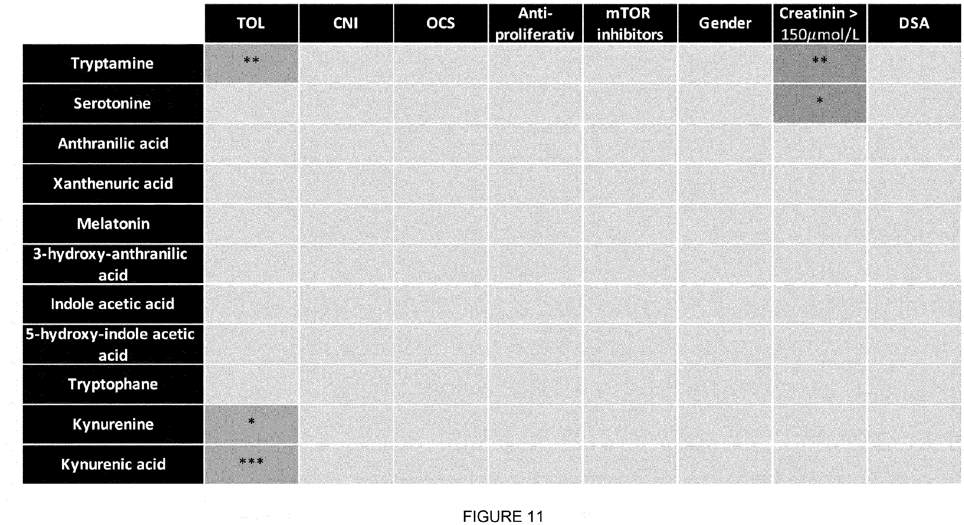

Figure 11 is a table representing the interaction matrix of the urinary tryptophan-derived metabolite in TOL detected thanks to RP UHPLC-MS method. Each column represents a tested factor among the interaction model (N-way ANOVA) for each of the twelve ions identified (in rows). A color code features either the upregulation (middle grey) or the downregulation (dark grey) or the absence of change (light grey) induced by the considered factor. Only tryptamine and serotonin were downregulated in the case of serum creatinine > 150*µ*mol/L. No interaction with immunosuppressive drugs was detected. The statistical significance of the interaction is represented by * indicating an FDR-adjusted p-value < 0.1 ; ** indicating an FDR-adjusted p-value < 0.01 and *** indicating an FDR-adjusted p-value < 0.001.

### EXAMPLES

### EXAMPLE 1 : MATERIAL & METHODS

### Patient selection and clinical data

Fifty-six patients were enrolled in the study and signed informed consent forms. The study groups were defined as follows: (1) sixteen spontaneously tolerant patients (TOL) with no immunosuppression for at least 1 year as previously described [13,14,16]; 10 patients with stable kidney graft function with creatinine <150 µmol/L and proteinuria <1 g/24 h plus six TOL with higher creatinine serum and/or proteinuria (150-402 µmol/Land proteinuria 1g-1.76g/24 h) without donor specific antibodies (DSA) which was attributed to vascular nephropathy by two-independent nephrologists. Thirteen patients with stable kidney graft function (creatinine <150 µmol/L and proteinuria <1 g/24 h) for at least 3 years under standard immunosuppression (calcineurin inhibitors (CNI), anti-metabolite ± corticosteroids) matched for post-transplantation time were selected [23]: (2) eight had normal histology (STA) whereas (3) five patients had histology proven subclinical ABMR (scABMR), (4) Thirteen minimally immunosuppressed patients (MIS) as previously described [24]; ten with stable kidney graft function (creatinine <150 µmol/L and proteinuria <1 g/24 h) for at least 3 years under 1 immunosuppressive drug (anti-metabolite or corticosteroids) plus three patients with higher serum creatinine attributed to CNI toxicity by two independent nephrologists and with stable creatinine for at least 3 years (creatinine 150-406 µmol/L and proteinuria 1g-7.4 g/24 h), (5) Fourteen healthy volunteers (HV) matched for age at sampling (+/- 5 years) without any medical history of immunosuppressive (IS) drugs in the last 6 months and without autoimmune or inflammatory disease, urinary tract infection or kidney diseases (Table 1). All the clinical data were retrospectively extracted from the DIVAT Integralis database for kidney-grafted patients (https://integralis.chu-nantes.fr/Default.aspx).

### Ethics statement

This study was performed in accordance with the Declaration of Helsinki and was approved by the National French Ethics Committee (CPP) N°337/2002 "Characterization of operational tolerance in kidney transplanted recipients without immunosuppressive drugs." And DIVAT (Données lnformatisées et VAlidées en Transplantation) (www.divat.fr, French Research Ministry: RC12_0452, last agreement No. 13 334, No. CNIL for the cohort: 891735)). All participants enrolled in this study signed informed consent forms.

### Urine samples collection

Urine samples (one sample per KTR and HV) were collected from TOL, STA, MIS and HV subjects from 2003 to 2020. Samples were collected aseptically after genital disinfection and were frozen at -80°C within 1 hour of sampling.

### Urine samples preparation

Urine samples were thawed on ice, homogenized and centrifuged at 4°C at 750g for 5 minutes. Urine pH and optic density were measured. Samples were then normalized on optic density using a refractometer (Digital Urine Specific Gravity Refractometer, 4410 (PAL-IOS), Cole-Parmer, USA) with ultrapure HPLC qualified water (Sigma-Aldrich, Saint Quentin Fallavier, France) and ultrafiltrate to retain molecules smaller than 10kDA (VWR, Fontenay-sous-Bois, France). Internal deuterated standards (leucine-5,5,5-d3, L-tryptophan 2,3,3 d3, acide indole-2,4,5,6,7-d5-3-acétique et acide 1,14-tétradécanedioïque-d24) were added to each sample in order to assess intra- and intersample validity. They were purchased from Sigma-Aldrich (Saint Quentin Fallavier, France) and CDN Isotopes (Quebec, Canada) and prepared in ethanol at 10 ng/µl.

### Urine metabolomic analysis by UHPLC/MS

Normalized and 10-kDa-filtered urine samples were analyzed on ultrahigh performance liquid chromatography with high-resolution mass spectrometry (UHLPC/MS) with the same apparatus and methods described in Peng et al [25] and in Narduzzi et al [26] for reversed phase (RP) UHPLC/MS and Hydrophilic Interaction Liquid Chromatography (HILIC) UHPLC/MS respectively. Tacking advantage of the MS² capacities of the hybrid quadrupole-orbitrap (Q-Exactive^{™}) mass spectrometer (Thermo Fisher Scientific, Bremen, Germany) QC samples (ie. pooled samples) were analyzed, in ESI positive and ESI negative modes, with three cycles of iterative Data Dependent MS² [27]. Samples were analyzed at random with regular QC samples injection (every 5 samples), following LC-MS metabolomics guidelines [28].

### Metabolomic signature process (Figure 2)

Data processing was performed under the Galaxy environment platform workflow4Metabolomic.org (W4M) [29,30] in two steps. The first step consisted of transforming raw UHPLC/MS files into a data matrix containing all the identified ions in each sample. For this purpose, each peak of mass spectrum (= an ion) in each sample was individualized using centwave algorithm based on the centroid approximation according to their mass (m/z) and retention time (RT) ("peak picking") [29,31]. Then, the same peaks (= the same ions) were aligned and gathered across samples according to their m/z and RT ("peak grouping") [29,31]. After grouping, missing data (= undetected ions) for each sample were integrated again according to m/z and RT in order to detect and create a new peak if available ("peak filling"). Finally, the batch effect was assessed and corrected using QC samples as an internal standard with the loess algorithm [29,32,33] and ions presenting a coefficient of variation (CV) > 30% in QC samples were filtered from the final data matrix. This step was performed on both ESI-positive and ESI-negative files which are available on online repositories. The second step consisted of individualizing a specific urinary metabolomic signature for TOL from the data matrix obtained at the former step. To this aim, a sample metadata files containing anonymous clinical and biological datas were generated. OPLS-DA multivariate analyses were performed with ions' relative intensities and the group status (TOL, MIS, STA, scABMR and HV) as the response nominal variable in order to discriminate the most impacting ions for each tested condition which was defined by a variable importance in projection (VIP) score value > 0.8 [30,33-36]. Metabolomic signatures were then extracted from the more impacting ions using first non-parametric univariate analysis followed by a multivariate analysis with ions' relative intensities thanks to the Biosigner algorithm [37].

### Metabolites identification

To identify metabolites from the TOL urinary metabolomic signature both MS and MS² data were used. In the MS data, adduct and isotopologues were searched using CAMERA annotation package [30,38]. The MS² data generated on pooled samples with iterative data dependant MS² acquisitions (iDDA) [27] were processed with msPurity [39] package tools included in W4M. In brief, every ion detected in the first step of "metabolomic signature process" was searched in the UHPLC/MS² files. If at least one MS² spectra was recorded for an ion then the MS2 spectra was compared to several external public databases (MassBank https://massbank.eu/MassBank/, HMDB https://hmdb.ca and GNPS https://gnps.ucsd.edu) This process allowed to annotate compounds at putative level (level 2) according to Creek and al [40]. The same process was used to identify tryptophan-derived metabolites: tryptophan, serotonin, melatonin, tryptamine, kynurenine, kynurenic acid, anthranilic acid, 3-hydroxyanthranilic acid, 5-hydroxyindoleactetic acid, indoleactetic acid, xanthurenic acid at a putative level (level 2). Then, standards for those metabolites were purchased from from Sigma-Aldrich (Saint Quentin Fallavier, France). This allowed the comparison of retention time, MS² mass spectra and identification of those compounds without a doubt in LC-MS traces (level 1) according to Creek et al [40].

### Metabolite quantification

Targeted absolute quantification of tryptophan-derived metabolites was performed on urine samples using UHPLC-MS² technology. For quantification purpose, standard solutions for calibration curves at six levels including isotopically labelled internal standards will be provided for each metabolite. Before analysis, every urine sample was deproteinized (10kD filtration) and then extracted with a methanol based solvent mix. After extraction and ad hoc dilution, the extracts was analyzed using an EXIONLC Ultra High Pressure Liquid Chromatography System coupled in-line to a 6500+ Q-Trap LC/MS system (Sciex, Framingham, MA, USA). After acquisition utilizing Analyst software v1.7 (Sciex, Framingham, MA, USA), the data was processed with skyline software [41].

### Statistical analysis

Univariate group comparisons were performed using nonparametric tests (Wilcoxon for 2 groups and Kruskal-Wallis for 3 or more groups). Unsupervised and supervised multivariate analyses were performed with ions' relative intensities using PCA and (O)PLS-DA respectively. Heatmaps were generated using correlation clustering and the ward aggregation algorithm [33] and interaction models were performed using N-way ANOVA under the Workflow4Metabolomics plateform [30]. Receiver Operating Characteristic Curve (ROCC) comparing TOL to nonTOL were generated using GraphPad Prism software version 6.0 (GraphPad software, California). False discovery rate correction (FDR) was applied to the p-value in case of multiple testing [42]. Statistical significance was considered from alpha risk < 0.1.

### EXAMPLE 2 : RESULTS

### Clinical and biological data of KTR and controls

The clinical and biological data from the 4 groups of recipients (STA, TOL, MIS and scABMR) and from HV were compared using ANOVA analysis for independent variables. No significant difference (adjusted p-value <0.05) was found for age at sampling, sampling time, transplantation rank, proteinuria and urinary pH among the recipient groups. No significant difference in gender ratio was observed between STA, TOL, MIS whereas scABMR and HV had a higher proportion of females without statistical significance (p-value <0.05 "ANOVA")). Furthermore, serum creatinine was not significantly different among recipient groups whereas it was significantly lower in HV (p-value = 1,2×10⁻⁴ "ANOVA") (Table 1). Of important note, KTR groups could be considered as representative of their respective wider population since no difference were noted between the clinical and routine biological parameters of KTR groups with those consensually used to define TOL [13,14,16], MIS [14] and STA [23].

### The urine metabolome profile of spontaneous tolerant recipients differs from that of other recipients and healthy volunteers.

After processing UHPLC/MS raw data, positive (ESI+) and negative ionization (ESI-) mode chromatograms were obtained containing 2171 ions in ESI+ and 2681 ions in ESI- with different mean intensities across the 4 recipient groups and the control group. Metabolomics profiles extended from polar to apolar metabolites following the acetonitrile gradient (Figure 3a). To further assess whether or not the urinary metabolome structure differed according to each group, principal component analysis (PCA) was used with the first three components (PC) in both ionization modes. In ESI+, TOL and HV were found to cluster together apart from STA, MIS and scABMR in the three PC projections (Figures 3a & 3b). In ESI-, HV clustered apart from recipients 'groups in the three PCs. The four recipient groups clustered in roughly parallel planes going from TOL/MIS/STA/scABMR in the three PCs as well (Figures 3a & 3c) echoing blood transcriptomic and urine microbiota data [13,22]. Altogether these results suggest a specific metabolomic signature for TOL that is distinct from other KTR and HV.

### Spontaneous tolerant recipients exhibit a urine-specific metabolomic signature mostly enriched in a tryptophan-derived metabolite: kynurenic acid.

To identify a urine-specific metabolomic signature in TOL, ions whose RT was greater than or equal to 60 seconds were kept to avoid a high coelution rate between ions (Figure 3a). MIS and STA were grouped under non-spontaneous tolerant patients (nonTOL) since multivariate analysis (OPLS-DA) could not strictly identify impacting ions to differentiate MIS and STA as shown by the permutation diagram in both ESI+ and ESI- modes in the cohort (Figure 4a) probably linked to long-term stability and time post-transplantation matching. Despite partial clustering of scAMBR in metabolomic profiling (Figures 3b & 3c), this group was not considered for further analysis since it was impossible to identify impacting ions to differentiate them from STA and MIS in both modes in our cohort (Figure 4b) probably due to small group size and a lack of statistical power. Using that strategy, twelve ions were identified that allowed to discriminate TOL from nonTOL and HV among which, ten were upregulated and two were downregulated in TOL patients compared to nonTOL and HV (Figure 5a). There was no interaction between those ions and clinical parameters such as gender, serum creatinine > 150*µ*mol/L or the presence of DSA at sampling. Interestingly, two ions (M183T391 and M289T387) interacted with immunosuppressive drugs: M183T391 was significantly downregulated in the presence of CNI and mTOR inhibitors (FDR adjusted p-value = 0.03 and 0.008 respectively "N-way ANOVA") whereas M289T387 was upregulated in the presence of CNI, antiproliferative drugs and/or oral corticosteroids (OCS) (FDR adjusted p-value = 0.0009; 0.00005; 0.0004 respectively "N-way ANOVA") (Figure 6). Among those twelve ions, eight ions were not identified since neither isotopic patterns nor matches in public databases or in our in-house bank were found. Interestingly, four ions with the same RT were identified in both modes corresponding to kynurenic acid (M144T186, M188T186, M190T186, M191T186) with significantly higher intensity threshold (1,4 fold compared to HV and 1,9 compared to nonTOL) in TOL versus nonTOL (FDR adjusted p-value = 9.10⁻⁶ "Kruskal Wallis test") and HV (FDR adjusted p-values = 0.015 "Kruskal Wallis test"). The associations of these four ions using ROCC analysis allowed to specifically discriminate TOL from the nonTOL and HV with a high sensitivity (Se = 81%) and specificity (Sp = 95%) when considering the relative intensity threshold of 4,3×10⁷ (Figure 5b and Figure 7). Altogether, these results show that TOL exhibit a specific urinary metabolomic profile that is strongly driven by kynurenic acid echoing to tryptophan metabolism pathways independent of immunosuppressive drugs, eGFR and gender.

### The urinary tryptophan metabolome of spontaneous tolerant recipients was skewed toward kynurenine and tryptamine pathways.

Because of the involvement of the kynurenic acid and to provide an overview of the involvement of the different pathways of the tryptophan and its metabolites in TOL, nonTOL and HV, the following metabolites were identified in the cohort: tryptophan, serotonin, melatonin, tryptamine, kynurenine, kynurenic acid, anthranilic acid, 3-hydroxyanthranilic acid, 5-hydroxyindoleactetic acid, indole-actetic acid and xanthurenic acid. Using RP UHPLC/MS method, kynurenine, kynurenic acid and tryptamine were found to be upregulated in TOL compared to nonTOL and HV whereas the remaining metabolites were not different between the different groups (Figure 8 & Figure 9). Using HILIC UHPLC/MS method, kynurenic acid was confirmed to be significantly increased in TOL compared to HV and nonTOL (FDR-adjusted p value = 0.08 and 0.01 respectively). There was no difference in kynurenine or tryptophan. Tryptamine could not be detected. At last, there was a significant decrease in xanthurenic acid in both TOL and nonTOL compared to HV (FDR-adjusted p value = 0.02 and 0.05 respectively "Kruskal Wallis test") (Figure 10). At last, no interaction was highlighted between the tryptophan-derived metabolites and gender, immunosuppressive drugs or the presence of DSA at sampling (Figure 11). Altogether, these results suggest that kynurenine pathway (encompassing kynurenic acid) and tryptamine pathway are associated with and upregulated in spontaneous operational tolerance (SOT) or low risk of rejection (LRR) in KTR.

### List of references

1. Kim S-Y, Kim BK, Gwon M-R, Seong SJ, Ohk B, Kang WY, et al. Urinary metabolomic profiling for noninvasive diagnosis of acute T cell-mediated rejection after kidney transplantation. J Chromatogr B Analyt Technol Biomed Life Sci. 2019 Jun 15;1118-1119:157-63.
2. Bassi R, Niewczas MA, Biancone L, Bussolino S, Merugumala S, Tezza S, et al. Metabolomic Profiling in Individuals with a Failing Kidney Allograft. PLoS One. 2017;12(1):e0169077.
3. Landsberg A, Sharma A, Gibson IW, Rush D, Wishart DS, Blydt-Hansen TD. Non-invasive staging of chronic kidney allograft damage using urine metabolomic profiling. Pediatr Transplantation. 2018 Aug;22(5):e13226.
4. Blydt-Hansen TD, Sharma A, Gibson IW, Mandal R, Wishart DS. Urinary metabolomics for noninvasive detection of borderline and acute T cell-mediated rejection in children after kidney transplantation. Am J Transplant. 2014 Oct;14(10):2339-49.
5. Blydt-Hansen TD, Sharma A, Gibson IW, Wishart DS, Mandal R, Ho J, et al. Urinary Metabolomics for Noninvasive Detection of Antibody-Mediated Rejection in Children After Kidney Transplantation. Transplantation. 2017 Oct;101(10):2553-61.
6. Sigdel TK, Schroeder AW, Yang JYC, Sarwal RD, Liberto JM, Sarwal MM. Targeted Urine Metabolomics for Monitoring Renal Allograft Injury and Immunosuppression in Pediatric Patients. J Clin Med. 2020 Jul 22;9(8):2341.
7. Zhong Z, Wheeler MD, Li X, Froh M, Schemmer P, Yin M, et al. L-Glycine: a novel antiinflammatory, immunomodulatory, and cytoprotective agent. Curr Opin Clin Nutr Metab Care. 2003 Mar;6(2):229-40.
8. Coras R, Murillo-Saich JD, Guma M. Circulating Pro- and Anti-Inflammatory Metabolites and Its Potential Role in Rheumatoid Arthritis Pathogenesis. Cells. 2020 Mar 30;9(4):827.
9. Alving AS, Miller BF. a practical method for the measurement of glomerular filtration rate (inulin clearance): with an evaluation of the clinical significance of this determination. Archives of Internal Medicine. 1940 Aug 1;66(2):306-18.
10. Duranton F, Lundin U, Gayrard N, Mischak H, Aparicio M, Mourad G, et al. Plasma and urinary amino acid metabolomic profiling in patients with different levels of kidney function. Clin J Am Soc Nephrol. 2014 Jan;9(1):37-45.
11. Goek O-N, Prehn C, Sekula P, Römisch-Margl W, Döring A, Gieger C, et al. Metabolites associate with kidney function decline and incident chronic kidney disease in the general population. Nephrol Dial Transplant. 2013 Aug;28(8):2131-8.
12. Khamis MM, Adamko DJ, El-Aneed A. Mass spectrometric based approaches in urine metabolomics and biomarker discovery. Mass Spectrometry Reviews. 2017 Mar 1;36(2):115-34.
13. Brouard S, Mansfield E, Braud C, Li L, Giral M, Hsieh S, et al. Identification of a peripheral blood transcriptional biomarker panel associated with operational renal allograft tolerance. Proc Natl Acad Sci USA. 2007 Sep 25;104(39):15448-53.
14. Roussey-Kesler G, Giral M, Moreau A, Subra J-F, Legendre C, Noel C, et al. Clinical operational tolerance after kidney transplantation. Am J Transplant. 2006 Apr;6(4):736-46.
15. Lozano JJ, Pallier A, Martinez-Llordella M, Danger R, López M, Giral M, et al. Comparison of transcriptional and blood cell-phenotypic markers between operationally tolerant liver and kidney recipients. Am J Transplant. 2011 Sep; 11(9):1916-26.
16. Massart A, Pallier A, Pascual J, Viklicky O, Budde K, Spasovski G, et al. The DESCARTES-Nantes survey of kidney transplant recipients displaying clinical operational tolerance identifies 35 new tolerant patients and 34 almost tolerant patients. Nephrol Dial Transplant. 2016 Jun;31(6):1002-13.
17. Newell KA, Asare A, Kirk AD, Gisler TD, Bourcier K, Suthanthiran M, et al. Identification of a B cell signature associated with renal transplant tolerance in humans. J Clin Invest. 2010 Jun; 120(6):1836-47.
18. Chesneau M, Michel L, Dugast E, Chenouard A, Baron D, Pallier A, et al. Tolerant Kidney Transplant Patients Produce B Cells with Regulatory Properties. Journal of the American Society of Nephrology. 2015 Oct 1;26(10):2588-98.
19. Durand M, Dubois F, Dejou C, Durand E, Danger R, Chesneau M, et al. Increased degradation of ATP is driven by memory regulatory T cells in kidney transplantation tolerance. Kidney Int. 2018 May;93(5):1154-64.
20. Dugast E, David G, Oger R, Danger R, Judor J-P, Gagne K, et al. Broad Impairment of Natural Killer Cells from Operationally Tolerant Kidney Transplanted Patients. Front Immunol. 2017;8:1721.
21. Chenouard A, Chesneau M, Bui Nguyen L, Le Bot S, Cadoux M, Dugast E, et al. Renal Operational Tolerance Is Associated With a Defect of Blood Tfh Cells That Exhibit Impaired B Cell Help. Am J Transplant. 2017 Jun;17(6):1490-501.
22. Colas L, Mongodin EF, Montassier E, Chesneau M, Guerif P, Hittle L, et al. Unique and specific Proteobacteria diversity in urinary microbiota of tolerant kidney transplanted recipients. Am J Transplant. 2020 Jan;20(1):145-58.
23. Legendre C, Canaud G, Martinez F. Factors influencing long-term outcome after kidney transplantation. Transpl Int. 2014 Jan;27(1):19-27.
24. Brouard S, Dupont A, Giral M, Louis S, Lair D, Braudeau C, et al. Operationally tolerant and minimally immunosuppressed kidney recipients display strongly altered blood T-cell clonal regulation. Am J Transplant. 2005 Feb;5(2):330-40.
25. Peng T, Royer A-L, Guitton Y, Le Bizec B, Dervilly-Pinel G. Serum-based metabolomics characterization of pigs treated with ractopamine. Metabolomics. 2017 May 12;13(6):77.
26. Narduzzi L, Royer A-L, Bichon E, Guitton Y, Buisson C, Le Bizec B, et al. Ammonium Fluoride as Suitable Additive for HILIC-Based LC-HRMS Metabolomics. Metabolites. 2019 Nov 27;9(12):E292.
27. Koelmel JP, Kroeger NM, Gill EL, Ulmer CZ, Bowden JA, Patterson RE, et al. Expanding Lipidome Coverage Using LC-MS/MS (ultra-high-resolution mass spectrometry) Data-Dependent Acquisition with Automated Exclusion List Generation. J Am Soc Mass Spectrom. 2017 May;28(5):908-17.
28. Want EJ, Wilson ID, Gika H, Theodoridis G, Plumb RS, Shockcor J, et al. Global metabolic profiling procedures for urine using UPLC-MS. Nat Protoc. 2010 Jun;5(6):1005-18.
29. Giacomoni F, Le Corguillé G, Monsoor M, Landi M, Pericard P, Pétéra M, et al. Workflow4Metabolomics: a collaborative research infrastructure for computational metabolomics. Bioinformatics. 2015 May 1;31(9):1493-5.
30. Guitton Y, Tremblay-Franco M, Le Corguillé G, Martin J-F, Pétéra M, Roger-Mele P, et al. Create, run, share, publish, and reference your LC-MS, FIA-MS, GC-MS, and Nuclear Magnetic Resonance (NMR) data analysis workflows with the Workflow4Metabolomics 3.0 Galaxy online infrastructure for metabolomics. Int J Biochem Cell Biol. 2017 Dec;93:89-101.
31. Smith CA, Want EJ, O'Maille G, Abagyan R, Siuzdak G. XCMS: Processing Mass Spectrometry Data for Metabolite Profiling Using Nonlinear Peak Alignment, Matching, and Identification. Anal Chem. 2006 Feb 1;78(3):779-87.
32. Dunn WB, Broadhurst D, Begley P, Zelena E, Francis-Mclntyre S, Anderson N, et al. Procedures for large-scale metabolic profiling of serum and plasma using gas chromatography and liquid chromatography coupled to mass spectrometry. Nat Protoc. 2011 Jul;6(7):1060-83.
33. Thévenot EA, Roux A, Xu Y, Ezan E, Junot C. Analysis of the Human Adult Urinary Metabolome Variations with Age, Body Mass Index, and Gender by Implementing a Comprehensive Workflow for Univariate and OPLS Statistical Analyses. J Proteome Res. 2015 Aug 7;14(8):3322-35.
34. Galindo-Prieto B, Eriksson L, Trygg J. Variable influence on projection (VIP) for orthogonal projections to latent structures (OPLS). Journal of Chemometrics. 2014;28(8):623-32.
35. Brereton RG, Lloyd GR. Partial least squares discriminant analysis: taking the magic away. Journal of Chemometrics. 2014;28(4):213-25.
36. Wold S, Sjöström M, Eriksson L. PLS-regression: a basic tool of chemometrics. Chemometrics and Intelligent Laboratory Systems. 2001 Oct 28;58(2):109-30.
37. Rinaudo P, Boudah S, Junot C, Thévenot EA. biosigner: A New Method for the Discovery of Significant Molecular Signatures from Omics Data. Front Mol Biosci. 2016;3:26.
38. Kuhl C, Tautenhahn R, Böttcher C, Larson TR, Neumann S. CAMERA: an integrated strategy for compound spectra extraction and annotation of liquid chromatography/mass spectrometry data sets. Anal Chem. 2012 Jan 3;84(1):283-9.
39. Lawson TN, Weber RJM, Jones MR, Chetwynd AJ, Rodríguez-Blanco G, Di Guida R, et al. msPurity: Automated Evaluation of Precursor Ion Purity for Mass Spectrometry-Based Fragmentation in Metabolomics. Anal Chem. 2017 Feb 21;89(4):2432-9.
40. Creek DJ, Dunn WB, Fiehn O, Griffin JL, Hall RD, Lei Z, et al. Metabolite identification: are you sure? And how do your peers gauge your confidence? Metabolomics. 2014 Jun 1;10(3):350-3.
41. Adams KJ, Pratt B, Bose N, Dubois LG, St. John-Williams L, Kevin M. Perrott KM, et al. Skyline for Small Molecules: A Unifying Software Package for Quantitative Metabolomics. Journal of Proteome Research. 2020 April: 1447-58.
42. Benjamini Y, Hochberg Y. Controlling the False Discovery Rate: A Practical and Powerful Approach to Multiple Testing. Journal of the Royal Statistical Society Series B (Methodological). 1995;57(1):289-300.

## Claims

1. An *in vitro* method for determining a spontaneous operational tolerance (SOT) profile and/or low risk of rejection (LRR) in a kidney transplant recipient (KTR), said method comprising:
a) determining in a urine sample of said KTR the expression level of at least one tryptophan-derived metabolite in said sample;
b) comparing the expression level detected in step a) to a reference value of said at least one tryptophan-derived metabolite;
c) deducing from said comparison that the KTR has a SOT profile when the expression level of at least one tryptophan-derived metabolite detected in step a) is equal to or higher than the reference value of said at least one tryptophan-derived metabolite.

2. The method according to claim 1, wherein step a) detects the expression level of at least one tryptophan-derived metabolite chosen from the group consisting of kynurenic acid, kynurenine, and tryptamine.

3. The method according to claim 2, wherein step a) detects the expression level of kynurenic acid.

4. The method according to claim 2 or 3, wherein the detection of the expression level of kynurenic acid comprises the detection of one or more ions chosen from the group consisting of ions M144T186, M188T186, M190T186 and M191T186.

5. The method according to claim 2, wherein the detection of the expression level of kynurenine comprises the detection of ions M209T8 and/or M207T89.

6. The method according to claim 2, wherein the detection of the expression level of tryptamine comprises the detection of ions M144T259.

7. The method according to any one of claims 1 to 6, wherein the reference value of at least one tryptophan-derived metabolite in step b) is the expression level of said at least one tryptophan-derived metabolite in a urine sample of a KTR having a SOT or LRR profile.

8. A kit-of-parts for performing the method according to any one of claims 1 to 7, comprising means for determining the expression level of at least one tryptophan-derived metabolites chosen from the group consisting of kynurenic acid, kynurenine and tryptamine.

9. A kit-of-parts according to claim 8, wherein means for determining the expression level of kynurenic acid comprises means for the detection of one or more ions chosen from the group consisting of ions M144T186, M188T186, M190T186 and M191T186.

10. A kit-of-parts according to claim 8, wherein means for determining the expression level of kynurenine comprises means for the detection of ions M209T89 and/or M207T89.

11. A kit-of-parts according to claim 8, wherein means for determining the expression level of tryptamine comprises means for the detection of ions M144T259.
